**Europäisches Patentamt**

**European Patent Office**

**Office européen des brevets**

(11) Veröffentlichungsnummer: **0 248 247 B1**

## EUROPÄISCHE PATENTSCHRIFT

(12)

(45) Veröffentlichungstag der Patentschrift: **22.01.92**

(21) Anmeldenummer: **87107045.4**

(22) Anmeldetag: **15.05.87**

(51) Int. Cl.⁵: **A61F  2/06**, A61L 27/00

---

(54) **Künstliche Gefässwand.**

---

(30) Priorität: **02.06.86 CH 2219/86**

(43) Veröffentlichungstag der Anmeldung:
**09.12.87 Patentblatt  87/50**

(45) Bekanntmachung des Hinweises auf die
Patenterteilung:
**22.01.92 Patentblatt  92/04**

(84) Benannte Vertragsstaaten:
**AT BE DE ES FR GB IT NL SE**

(56) Entgegenhaltungen:
**EP-A- 0 130 401**
**EP-A- 0 157 178**
**EP-A- 0 183 365**
**WO-A-82/03764**

(73) Patentinhaber: **GEBRÜDER SULZER AKTIENGE-
SELLSCHAFT**
**Zürcherstrasse 9**
**CH-8401 Winterthur(CH)**

(72) Erfinder: **Turina, Marko, Prof. Dr.**
**In den Looren 40**
**CH-8053 Zürich(CH)**
Erfinder: **Bittmann, Peter, Dr.**
**Langackerstrasse 126**
**CH-8704 Herrliberg(CH)**

**Beschreibung**

Die Erfindung betrifft eine künstliche Gefässwand, insbesondere für arterielle Gefässe mit Innendurchmessern kleiner 7 mm, bestehend aus einer durchlässigen, mit durchgehend offenen Mikroporen versehenen Membran aus bioinertem Kunststoff.

Aus der Erfahrung ist bekannt, dass für Flüssigkeiten - vor allem für Wasser, Nährstoffe, Gewebeflüssigkeit und Stoffwechselprodukte - undurchlässige Substanzen als künstliche Gefässwände ungeeignet sind, da die Bewegung der Gewebeflüssigkeit dadurch weitgehend behindert wird, was zu Störungen des physiologischen Gleichgewichts und zu heftigen Reaktionen des Körpers führt. Es ist daher zu fordern, dass künstliche Gefässwände für Flüssigkeiten mindestens teildurchlässig sind.

Ein Arterienersatz im Bereich grosser Gefässe - beispielsweise der Aorta bis zur Leistengegend - gilt als weitgehend gelöst, beispielsweise durch textile Prothesen oder durch Prothesen aus expandiertem Polytetrafluoräthylen.

Bei diesen "grossen" Gefässen wird die erwähnte Durchlässigkeit der Gefässwand durch den textilen Aufbau oder durch den Reckprozess erreicht, wobei gleichzeitig durch von aussen einwachsendes Gewebe eine Fixierung erzielt wird.

Kleinlumige Gefässprothesen der genannten Art mit einem Innendurchmesser unterhalb 7 mm sind bekannt aus der EP-A-0 130 401; sie sind blutstromseitig mit einer sogenannten "skin layer" versehen, in der Poren von 1 bis 50 um zur Bildung und Stabilisierung von "Pseudointima" vorhanden sind. Pseudointima besteht bekanntlich aus "toten" Blutbestandteilen; sie kann bis zu Schichtdicken von etwa 1 mm wachsen. Künstliche kleinlumige Gefässe neigen daher zum Verschluss. Durch das dadurch verlangsamt fliessende Blut wird eine weitere Ablagerung begünstigt und es kommt zur Thrombosierung der Prothese. Zur Ueberwindung dieser Schwierigkeit sind Glandula-Prothesen bekannt (DE-PS 34 22 639), bei denen vor der Implantation die Innenseite mit einer Monoschicht patienteneigener Endothel-Zellen lückenlos ausgekleidet ist.

Aus der WO-A-82/03764 ist es bekannt Endothel-Zellen auf lebendem Zellgewebe, beispielsweise auf glatten Muskelzellen (SMC) zu züchten.

Eine ausreichende Durchlässigkeit haben an sich flüssigkeitsdurchlässige Gele - beispielsweise wässrige Gele auf Agar-Agar- oder Polyacrylamid-Basis -. Diese Gele sind als Gefässwände jedoch ungeeignet, da sie keine ausreichende mechanische Festigkeit haben, die beispielsweise den Druckpulsationen arterieller Blutströme zu widerstehen vermag.

Für derartige Gefässwände besitzen jedoch Kunststoffmembranen die notwendige mechanische Stabilität; ihre Durchlässigkeit wird durch durchgehend offene Poren gewährleistet. Da bei ihnen gleichzeitig eine Fixierung durch aussen ein- oder anwachsendes Gewebe erreicht werden soll, sind Porengrösse von mindestens 10 bis 50 $\mu$, vorzugsweise 30 $\mu$, zu fordern.

Für die Haftung und Lebensfähigkeit einer Monoschicht aus Endothel-Zellen ist es notwendig, dass die Endothel-Zellen einen geschlossenen zusammenhängenden Belag bilden. Ihre Haftung auf dem Kunststoff, auf dem sie unter Bildung einer Basalmembran aus Kollagen, Typ IV, wachsen, muss dabei so gross sein, dass sie vom Blutstrom nicht mitgerissen werden. Bei Verwendung von Kunststoff-Membranen, deren Porengrösse die genannten Werte erreichen, hat sich nun gezeigt, dass derart grosse "Löcher" in der Kunststoffoberfläche von den Endothel-Zellen nicht überwachsen werden können, so dass sich eine geschlossene Schicht von Endothel-Zellen nicht bildet.

Aufgabe der Erfindung ist es, eine mechanisch stabile Gefässwand für kleinlumige Gefässe zu schaffen auf der die Bildung, Haftung und Lebensfähigkeit einer einlagigen Schicht aus Endothel-Zellen gewährleistet ist. Die Lösung dieser Aufgabe erfolgt zum einen dadurch, dass die Membran mindestens auf ihrer blutstromseitigen Oberfläche derart geglättet ist, dass einerseits ihre Permeabilität mindestens teilweise erhalten bleibt, andererseits jedoch eine geschlossene Monoschicht von Endothel-Zellen wächst und die Membran blutstromseitig einlagig mit Endothel-Zellen belegt ist, wobei die Mikroporen zur Glättung der Membranoberfläche mit einem wässrigen Gel gefüllt sind, das für Moleküle mit einem Molekulargewicht bis zu 100'000 u (atomare Masseneinheiten (Dalton) permeabel ist. Zum anderen kann diese Aufgabe dadurch gelöst werden, dass mindestens die blutstromseitigen Oberflächen der Mikroporen mit einer porösen Schicht geschlossen sind, bei der der Durchmesser der Poren mindestens annähernd mit den Abmessungen von Endothel-Zellen vergleichbar ist.

Durch das Füllen oder Abdecken der "grossen" Poren wird die Oberfläche der Membran mindestens auf ihrer Innenseite geglättet, so dass die Endothel-Zellen die Störstellen der Oberfläche überwachsen können, so dass sie eine geschlossene zusammenhängende Monoschicht bilden.

Als wässrige Gele für das Füllen der Poren eignen sich beispielsweise die bereits erwähnten Agar-Agar- oder Polyacrylamidgele. Für das Abdecken der "grossen" Poren mit Membranen, bei denen der Durchmesser der Poren mindestens annähernd mit demjenigen von Endothel-Zellen vergleichbar ist, lassen sich beispielsweise Membranen verwenden, wie sie aus dem Gebiet der Ultrafiltration bekannt sind.

Vorteilhaft ist es dabei, wenn die Aussenfläche der Membran zusätzlich mit Zellen einer glatten elastische Fasern erzeugenden Muskulatur (SMC: smooth muscle cells) beschichtet ist; zwischen den Endothel-Zellern und den Muskelzellen, die ein- oder mehrschichtig - gleichzeitig mit den Endothel-Zellen oder in getrennten Arbeitsgängen - auf die äussere Oberfläche der Membran aufgebracht sein können, ergeben sich so Wechselwirkungen biochemischer und physiologischer Art, die zu einer Verbesserung der Lebensfähigkeit der lebenden Zellen auf der Kunststoffmembran führen. Die von den Muskelzellen gebildeten elastischen Fasern verstärken darüberhinaus die Membran als Gefässwand und sichern die Elastizität auch wenn der Kunststoff der Membran langzeitig unter Umständen Veränderungen erleidet.

Die bioinerte Kunststoff-Membran besteht vorteilhaft aus einem Polyurethan von 0,2 bis 1 mm Dicke, wobei die Durchmesser der Mikroporen mindestens teilweise zwischen 10 und 50 $\mu$m, vor-zugsweise bei 30 $\mu$m, liegen können. Solche Mem-branen können beispielsweise nach dem bekannten Verfahren der Phasentrennung bei niedrigen Temperaturen hergestellt werden.

Natürliche Gefässwände sind bekanntlich so ausgebildet, dass sie bei Erhöhungen des Innendrucks eine elastische Dehnung aufweisen, die mit steigender Druckerhöhung einem oberen Grenzwert zustrebt. Kunststoffmembranen besitzen diese Eigenschaften des natürlichen Gewebes nur unvollkommen. Es ist daher zweckmässig, die Dehnbarkeit der Membran mit Hilfe einer stützenden Struktur zu begrenzen, die vorteilhafterweise aus textiler Maschenware besteht, deren Maschenweite zwischen 0,2 und 2 mm beträgt. Dabei hat sich besonders eine stützende Struktur aus einem Geflecht einzelner spiralförmig angeordneter Monofilamente aus Polyester-Fäden von 10 bis 40 $\mu$m Durchmes-ser bewährt. Das Geflecht, dessen einzelne Filamente praktisch unelastisch sind, erhält eine gewisse Elastizität durch den textilen Aufbau, bei dem sich die einzelnen Filamente bei Druckänderungen relativ zueinander umlagern. In diesem Zusammenhang ist durch experimentelle Versuche ermittelt worden, dass die lineare Dehnbarkeit der künstlichen Gefässwand in einem Druckbereich des Innendruckes von 1 bis 2 $\cdot$ 10$^4$ Pa (80 bis 150 mmHg) mit Vorteil 0,04 bis 0,13 % pro 100 Pa (0,03 bis 0,1 % pro mmHg) Druckerhöhung betragen kann. Der spiralförmige Aufbau des Geflechtes ist knicksicher und verleiht so der Membran eine erhöhte Knickstabilität.

Im folgenden wird die Erfindung anhand eines Ausführungsbeispiels im Zusammenhang mit der Zeichnung näher erläutert.

Fig. 1     zeigt schematisch und vergrössert - ohne dass die einzelnen Elemente massstabgerecht zueinander dargestellt sind - einen Schnitt durch eine erste Ausführungsform der neuen Gefässwand;

Fig. 2     ist ein zweites Ausführungsbeispiel, bei dem - gegenüber Fig. 1 nochmals vergrössert - ein Bereich an der Innenseite der neuen Gefässwand im Schnitt dargestellt ist.

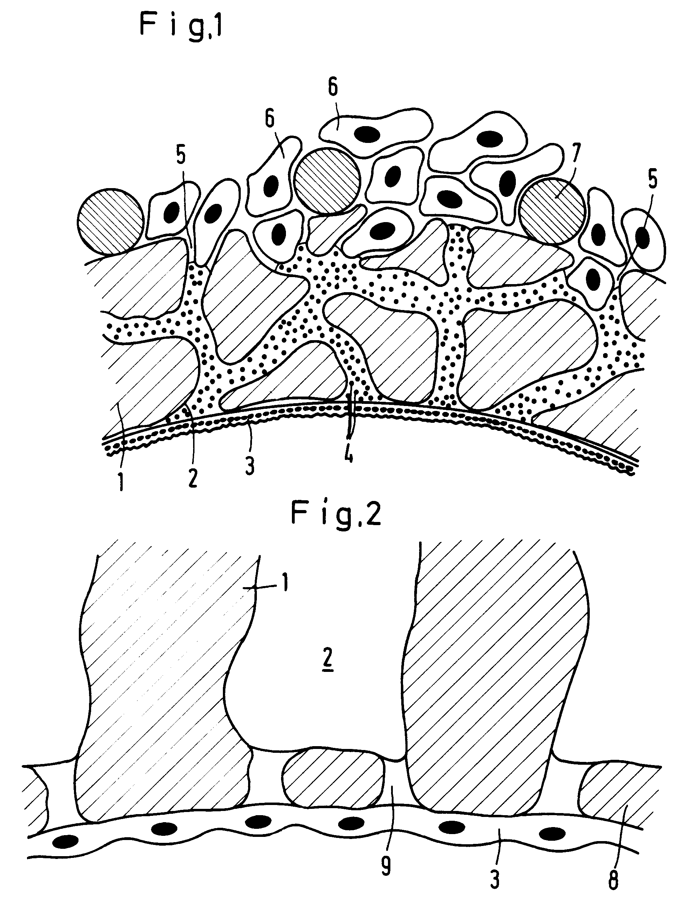

Das "Wandelement" der neuen Gefässwand bildet eine mikroporöse Kunststoff-Membran 1 (Fig. 1), die vorzugsweise aus einem Polyurethan besteht und eine Dicke zwischen 0,2 und 1 mm hat. In ihr ist ein Netz von Mikroporen 2 vorhanden, deren Durchmesser zumindest vorwiegend zwi-schen 10 und 50 $\mu$m betragen. Wie Fig. 1 zeigt, sind die Mikroporen 2 sowohl an der äusseren als auch an der inneren Oberfläche der Membran 2 offen.

Die innere Oberfläche der Membran 1 ist mit einer Monoschicht 3 von lebenden Endothel-Zellen belegt. Da diese - wie geschildert - Hohlräume, deren Durchmesser ein Mehrfaches ihrer eigenen Abmessungen betragen, im allgemeinen nicht oder nur schwer überwachsen können, ist die innere Oberfläche der Membran 1 "geglättet". Bei der ersten Ausführungsform erfolgt diese Glättung durch eine Füllung der Mikroporen 2 mit einem, die Permeabilität der Membran 1 nicht unzulässig beeinträchtigenden wässrigen Gel 4. Dieses Gel 4, das beispielsweise ein 6%-iges Agarose- oder ein 12 bis 18%-iges Polyacrylamidgel sein kann, wird beispielsweise nach einem bekannten Tauchverfahren in die Mikroporen 2 eingefüllt. Nach dem Tauchen wird es an der äusseren Oberfläche der Membran 1 teilweise wieder herausgelöst, um Hohlräume 5 zu schaffen, in die - zur Fixierung des künstlichen Gefässes im Körper - Zellen 6 einwachsen können.

Die Zellen 6 bestehen erfindungsgemäss aus glatten Muskelzellen 6 (SMC), die ebenfalls auf bekannte Weise gezüchtet und auf die Membran oder Gefässwand 1 ein- oder mehrschichtig aufgebracht werden. Diese glatten Muskelzellen haben einerseits die Aufgabe, durch physiologische und biochemische Wechselwirkungen durch die Membran 1 hindurch die "Verträglichkeit" des künstlichen Gefässes im Körper zu fördern; zum anderen bilden sie elastische Muskelfasern, die die mechanische Stabilität des künstlichen Gefässes erhöhen. Diese Wechselwirkungen werden ausser durch die Durchlässigkeit auch durch die Elastizität der Kunststoffmembran 1 ermöglicht, die die Druckimpulskurven des Blutstromes an die Muskelzellen 6 weitergibt und ihre Produktion elastischer Fasern (Elastin) anregt.

Wie bereits erwähnt, ist die Dehnbarkeit natürlicher Gefässe und diejenige der Endothel-Zellen begrenzt; um eine ähnliche Begrenzung der Dehnung für eine mit festhaftenden Endothel-Zellen belegte künstliche Gefässwand aus einer Kunststoffmembran 1 zu erreichen, sind über die äussere Oberfläche der Membran 1 als stützende, nur wenig elastische Struktur einzelne Fäden 7 verteilt. Diese Fäden 7 sind beispielsweise unelastische Monofilamente aus Polyester mit Durchmesser von 10 bis 40 μm. Für ein in sich peripher geschlossenes aderartiges künstliches Gefäss besteht die Stützstruktur der Fäden 7 beispielsweise aus einem Geflecht, dessen Monofilamente spiralförmig verlaufen und das seine begrenzte Elastizität aufgrund der mechanischen Verschiebbarkeit der einzelnen Fäden 7 erhält. Die erwähnten Dicken der Fäden 7 und Maschenweiten des Geflechtes von beispielsweise 0,2 bis 2 mm ermöglichen es, die lineare Dehnung bei einem Innendruck im physiologischen Bereich zwischen 80 bis 150 mmHg auf 0,03 bis 0,1 % por mmHg Druckerhöhung zu begrenzen.

Die Herstellung der "Füll"-Gele kann durch verschiedene miteinander kombinierte bekannte Verfahrensschritte erfolgen. Im Rahmen dieser bekannten Verfahren wird das Agarose-Gel darüberhinaus in ebenfalls bekannter Weise im Hinblick auf ein verbessertes Anwachsen von lebenden Zellen durch eine elektrische Oberflächenbeladung konditioniert.

Eine Sterilisation der zu implantierenden Gefässwand bzw. ihrer Einzelteile erfolgt beispielweise auf chemischem Weg durch Einlegen in einer Sterilisationslösung.

Das Wachstum der Endothel-Zellen 3 über die Mikroporen 2 wird bei dem Ausführungsbeispiel nach Fig. 2 durch eine dünne Schicht 8 erreicht, deren Dicke ein Zehntel bis ein Hundertstel der Dicke der Membran 1 beträgt; mit Vorteil, jedoch nicht zwingend, kann diese Schicht 8 aus dem Grundmaterial der Membran 1 bestehen. Die Schicht 8 hat Poren 9, deren Durchmesser mit den Abmessungen der Endothel-Zellen 3 vergleichbar sind, die mehrere Mikrometer μm, beispielsweise 3 bis 10 μm, betragen. Die Herstellung derartiger, relativ zur Membran 1 kleinporiger Membranschichten 8 ist aus der Technik der Ultrafiltration bekannt; weiterhin ist die Herstellung von Membranen, bei denen die Porengrösse gezielt in einer Richtung wächst, mit Hilfe der bekannten Verfahren der Phasentrennung bzw. Phaseninversion möglich.

**Patentansprüche**

1. Künstliche Gefässwand, insbesondere für arterielle Gefässe mit Innendurchmessern kleiner 7 mm, bestehend aus einer durchlässigen, mit durchgehend offenen Mikroporen (2) versehenen Membran (1) aus einem bioinerten Kunststoff, dadurch gekennzeichnet, dass die Membran (1) mindestens auf ihrer blutstromseitigen Oberfläche derart geglättet ist, dass einerseits ihre Permeabilität mindestens teilweise erhalten bleibt, andererseits jedoch eine geschlossene Monoschicht von Endothel-Zellen (3) wächst und die Membran blutstromseitig einlagig mit Endothel-Zellen belegt ist, wobei die Mikroporen (2) zur Glättung der Membranoberfläche mit einem wässrigen Gel (4) gefüllt sind, das für Moleküle mit einem Molekulargewicht bis zu 100'000 u (atomare Masseneinheiten (Dalton)) permeabel ist.

2. Künstliche Gefässwand, insbesondere für arterielle Gefässe mit Innendurchmessern kleiner 7 mm, bestehend aus einer durchlässigen, mit durchgehend offenen Mikroporen (2) versehenen Membran (1) aus einem bioinerten Kunststoff, dadurch gekennzeichnet, dass die Membran (1) mindestens auf ihrer blutstromseitigen Oberfläche derart geglättet ist, dass einerseits ihre Permeabilität mindestens teilweise erhalten bleibt, andererseits jedoch eine geschlossene Monoschicht von Endothel-Zellen (3) wächst und die Membran blutstromseitig einlagig mit Endothel-Zellen belegt ist, wobei mindestens die blutstromseitigen Oberflächen der Mikroporen (2) mit einer porösen Schicht (8) geschlossen sind, bei der der Durchmesser der Poren (9) mindestens annähernd mit den Abmessungen von Endothel-Zellen (3) vergleichbar ist.

3. Gefässwand nach Anspruch 1 oder 2, dadurch gekennzeichnet, dass die Aussenfläche der Membran (1) mit Zellen (6) einer glatten, elastische Fasern erzeugenden Muskulatur (SMC) beschichtet ist.

4. Gefässwand nach Anspruch 1 oder 2, dadurch gekennzeichnet, dass die Kunststoff-Membran (1) aus einem Polyurethan von 0,1 bis 1 mm Dicke besteht.

5. Gefässwand nach Anspruch 1, dadurch gekennzeichnet, dass die Durchmesser der Mikroporen (2) mindestens teilweise zwischen 10 und 50 μm, vorzugsweise bei 30 μm, liegen.

6. Gefässwand nach Anspruch 1 oder 2, dadurch gekennzeichnet, dass die Dehnbarkeit der Membran (1) mit Hilfe einer stützenden Struktur (7) begrenzt ist.

7. Gefässwand nach Anspruch 6, dadurch gekennzeichnet, dass bei einem Innendruck von

1 bis 2 $\cdot$ 10$^4$ Pa (80 bis 150 mm Hg) die lineare Dehnung der Membran (1) 0,04 bis 0,13 % pro 100 Pa (0,03 bis 0,1 % pro mm Hg) Druckerhöhung beträgt.

8. Gefässwand nach Anspruch 6 oder 7, dadurch gekennzeichnet, dass die stützende Struktur (7) aus textiler Maschenware besteht, deren Maschenweite zwischen 0,2 und 2 mm beträgt.

9. Gefässwand nach Anspruch 9, dadurch gekennzeichnet, dass die stützende Struktur (7) aus einem Geflecht einzelner spiralförmig angeordneter Filamente aus Polyester-Fäden von 10 bis 40 $\mu$m Durchmesser besteht.

## Claims

1. An artificial vessel wall, more particularly for arterial vessels of less than 7 mm internal diameter, the wall comprising a permeable membrane (1) which is made of a bio-inert plastic and which is formed with continuously open micropores (2), characterised in that the membrane (1) is so smoothed at least on its surface on the blood flow side that its permeability remains at least to some extent but a closed monolayer of endothelial cells (3) grows and the membrane is covered on the blood flow side with a single layer of endothelial cells, the micropores (2) being filled with an aqueous gel (4) to smooth the membrane surface, such gel being permeable for molecules having a molecular weight of up to 100 000 u (atomic mass units (Dalton)).

2. An artificial vessel wall, more particularly for arterial vessels of less than 7 mm internal diameter, the wall comprising a permeable membrane (1) which is made of a bio-inert plastic and which is formed with continuously open micropores (2), characterised in that the membrane (1) is so smoothed at least on its surface on the blood flow side that its permeability remains at least to some extent but a closed monolayer of endothelial cells (3) grows and the membrane is covered on the blood flow side with a single layer of endothelial cells, and the micropore surfaces on the blood flow side are closed by a porous layer (8) in which the diameter of the pores (9) is comparable at least approximately to the dimensions of endothelial cells (3).

3. A vessel wall according to claim 1 or 2, characterised in that the outside surface of the membrane (1) is coated with smooth muscle cells (SMC) (6) which produce elastic fibres.

4. A vessel wall according to claim 1 or 2, characterised in that the plastic membrane (1) is made of a 0.1 to 1 mm thick polyurethane.

5. A vessel wall according to claim 1, characterised in that the diameters of the micropores (2) are at least to some extent between 10 and 50 $\mu$m, preferably 30 $\mu$m.

6. A vessel wall according to claim 1 or 2, characterised in that the stretch of the membrane (1) is limited by means of a support structure (7).

7. A vessel wall according to claim 6, characterised in that in response to an internal pressure of from 1 to 2 $\cdot$ 10$^4$ Pa (80 to 150 mm Hg) the linear stretch of the membrane (1) is from 0.04 to 0.13% per 100 Pa (0.03 to 0.1%/mm Hg) of pressure increase.

8. A vessel wall according to claim 6 or 7, characterised in that the support structure (7) consists of a textile fabric having a mesh size between 0.2 and 2 mm.

9. A vessel wall according to claim 9, characterised in that the support structure (7) consists of a braiding of discrete spiral filaments of polyester threads of from 10 to 40 $\mu$m diameter.

## Revendications

1. Paroi artificielle de vaisseau, en particulier pour des vaisseaux artériels ayant un diamètre intérieur qui est inférieur à 7 mm, se composant d'une membrane perméable (1) de matière plastique biologiquement inerte qui comporte des micropores (2) présentant une ouverture continue, caractérisée en ce que la membrane (1) est lissée au moins à la surface qui est du côté du flux sanguin de manière d'une part que sa perméabilité soit au moins partiellement conservée, mais d'autre part de manière qu'une monocouche fermée de cellules endothéliales (3) se forme par croissance et que la membrane soit revêtue du côté du flux sanguin d'une monocouche de cellules endothéliales, les micropores (2) étant remplis, pour le lissage de la surface de la membrane, d'un gel aqueux (4) qui est perméable pour des molécules ayant un poids moléculaire pouvant atteindre 100 000 u (unités atomiques de masse (Dalton)).

2. Paroi artificielle de vaisseau, en particulier pour vaisseaux artériels ayant des diamètres intérieurs qui sont inférieurs à 7 mm, se composant d'une membrane perméable (1) de matière plastique biologiquement inerte qui comporte des micropores (2) présentant une ouverture continue, caractérisée en ce que la membrane (1) est lissée au moins à la surface située du côté du flux sanguin de manière que d'une part sa perméabilité soit au moins partiellement conservée, mais d'autre part de manière qu'une monocouche fermée de cellules endothéliales (3) se forme par croissance et que la membrane soit revêtue du côté du flux sanguin d'une monocouche de cellules endothéliales, au moins les surfaces des micropores (2) qui sont tournées du côté du flux sanguin étant fermées par une couche poreuse (8) dont le diamètre des pores (9) est au moins approximativement comparable aux dimensions des cellules endothéliales (3).

3. Paroi de vaisseau selon la revendication 1 ou 2, caractérisée en ce que la surface extérieure de la membrane (1) est revêtue de cellules (6) de tissu musculaire lisse, produisant des fibres élastiques (SMC).

4. Paroi de vaisseau selon la revendication 1 ou 2, caractérisée en ce que la membrane de matière plastique (1) est en un polyuréthanne de 0,1 à 1 mm d'épaisseur.

5. Paroi de vaisseau selon la revendication 1, caractérisée en ce que le diamètre des micropores (2) est compris au moins en partie entre 10 et 50 $\mu$m et il est de préférence de 30 $\mu$m.

6. Paroi de vaisseau selon la revendication 1 ou 2, caractérisée en ce que l'extensibilité de la membrane (1) est limitée à l'aide d'une structure de soutien (7).

7. Paroi de vaisseau selon la revendication 6, caractérisée en ce que l'extension linéaire de la membrane (1) est de 0,04 à 0,13 % par 100 Pa (0,03 à 0,1 % par mm de Hg) d'élévation de pression pour une pression interne de 1 à 2.10$^4$ Pa (80 à 150 mm de Hg).

8. Paroi de vaisseau selon la revendication 6 ou 7, caractérisée en ce que la structure de soutien (7) consiste en un produit textile à mailles dont l'ouverture des mailles est comprise entre 0,2 et 2 mm.

9. Paroi de vaisseau selon la revendication 9, caractérisée en ce que la structure de soutien (7) se compose d'une entrelacement de filaments individuels disposés en hélice et formés de fils de polyester de 10 à 40 $\mu$m de diamètre.

# F i g.1

# F i g.2